# EUROPEAN PATENT SPECIFICATION

(11) **EP 4 228 435 B1**
(45) Date of publication and mention of the grant of the patent: **28.01.2026**
(21) Application number: 21786969.2
(22) Date of filing: 07.10.2021
(51) Int. Cl.: A23L 33/00, A61K 31/702, A61P 1/00

(54) **INFANT OR YOUNG CHILD FORMULA**
SÄUGLINGSNAHRUNG ODER KLEINKINDERNAHRUNG
FORMULE POUR NOURRISSONS OU JEUNE ENFANT

(30) Priority: 16.10.2020 EP 20202255
(43) Date of publication of application: 23.08.2023
(73) Proprietor: Société des Produits Nestlé S.A., 1800 Vevey (CH)
(72) Inventor: HEINE, Ralf, Günter, 10711 Berlin (DE)
(74) Representative: Elkington and Fife LLP
(86) International application number: PCT/EP2021/077656
(87) International publication number: WO 2022/078861

(56) References cited:
- WO-A1-2016/139328
- WO-A1-2016/139333
- WO-A1-2017/021476
- WO-A1-2021/074375
- US-A1- 2018 333 426
- US-A1- 2019 209 593
- MARLENE BORSCHEL ET AL: "Comparison of Growth of Healthy Term Infants Fed Extensively Hydrolyzed Protein- and Amino Acid-Based Infant Formulas", NUTRIENTS, vol. 10, no. 3, 1 March 2018 (2018-03-01), pages 289, XP055661829, DOI: 10.3390/nu10030289
- EUROPEAN FOOD SAFETY AUTHORITY: "Safety of lacto- N -neotetraose as a novel food ingredient pursuant to Regulation (EC) No 258/97", THE EFSA JOURNAL, vol. 13, no. 7, 1 July 2015 (2015-07-01), Parma, IT, pages 4183 - 1, XP055364478, ISSN: 1831-4732, DOI: 10.2903/j.efsa.2015.4183
- L. BODE: "Human milk oligosaccharides: Every baby needs a sugar mama", GLYCOBIOLOGY, vol. 22, no. 9, 18 April 2012 (2012-04-18), pages 1147 - 1162, XP055199362, ISSN: 0959-6658, DOI: 10.1093/glycob/cws074

## Description

### FIELD OF THE INVENTION

The present invention relates to nutritional compositions for infants and young children and their health effects in infants. In particular, it relates to infant or young child formula comprising the human milk oligosaccharides (HMOs) 2'-fucosyllactose (2FL) and lacto-N-neotetraose (LNnT), for modulating maturation of the gut microbiome. The formula is an extensively hydrolysed formula (eHFs) or an amino acid-based infant formula (AAFs) and is used in an infant with cow's milk protein allergy.

### BACKGROUND TO THE INVENTION

Cow's milk protein (CMP) is the leading cause of food allergy in infants, affecting 2-3% children worldwide. Most children with CMP-allergy (CMPA) have two or more symptoms: 50-70% have skin symptoms; 50-60% have gastrointestinal symptoms; and 20-30% have airway symptoms. Severe and life-threatening symptoms may occur in 10% of children. (Nutten, 2018. EMJ Allergy Immunol, 3(1), pp. 50-59).

Human breast milk and breast feeding are considered to be the optimal form of nutrition for healthy infants during the first months of life. Breast milk remains the gold standard for feeding infants with CMPA. The European Society for Paediatric Gastroenterology, Hepatology and Nutrition (ESPGHAN) recommends that CMPA is best treated in breast-fed infants by complete elimination of cow's milk from the mother's diet (Koletzko, S., et al., 2012. Journal of Pediatric Gastroenterology and Nutrition, 55(2), pp.221-229).

Specialty infant formulas are recommended when breastfeeding is not possible. ESPGHAN recommends that for non-breast-fed infants with CMPA, formulas based on extensively hydrolysed proteins (eHF) are used, with proven efficacy in infants with CMPA. In infants with severe or life-threatening symptoms, an amino acid-based infant formula (AAF) may be considered as the first choice (Koletzko, S., et al., 2012. Journal of Pediatric Gastroenterology and Nutrition, 55(2), pp.221-229).

There is growing evidence regarding the role of infant gut microbial composition in the immune trajectory and allergy development of the infant host (Quante M. et al. (2012) BMC Public Health 12: 1021). As such, environmental factors such as diet, pollution, urban lifestyle, cleanliness and birth method have been associated with the development of the immune system and allergic diseases (Seppo, A.E. et al. (2017) J Allergy Clin Immunol 139: 708-11 e5; Azad, M.B. et al. (2018) J Nutr 148: 1733-42).

Infancy, especially the first weeks, 3 months, 6 months or 12 months of life is a critical period for the establishment of a balanced gut microbiota.

It is known that the modulation of the gut microbiota during infancy can prospectively have a significant influence in the future health status of the body. For example, the gut microbiome can influence the development of a strong immune system later in life, as well as normal growth, and even on the development of obesity later in life.

The gut microbiome and its evolution during the development of the infant is, however, a fine balance between the presence and prevalence (amount) of many populations of gut bacteria. Some gut bacteria are classified as "generally positive" while others are "generally negative" (or pathogenic) regarding their effect on the overall health of the infant. Certain species of "generally positive" bacteria, such as bifidobacteria, may be under-represented in infants fed conventional infant formula in comparison to breastfed infants. Similarly, some bacterial populations are considered pathogenic and should remain at a low prevalence in the gut microbiota.

Infants fed infant formulae may not benefit from the healthy, well balanced intestinal gut microbiome seen in infants fed exclusively, or predominantly, human breast milk. The development of a healthy microbiome in the first years of life is complex and prone to disturbances by environmental factors. Many taxa of micro-organisms co-exist in the highly complex microenvironment of the gut/intestine, each in sequentially defined proportions. Quantitative and qualitative dimensions are to be considered when defining the microbiota of infants or young children. Furthermore, the variation over time of the gut microbiota adds to the complexity. The fine balance of all the families, genera, species and strains of bacteria present in each location of the gastrointestinal tract, as well as their variation over time, all contribute to the "gastro-intestinal health" of infants and young children.

Recent studies have observed a consistent increase in microbiota age for breast-fed infants compared to formula-fed infants, receiving little or no breast milk. These early changes in gut microbiota associated with formula-fed infants have been inversely linked to immunological and biological maturity of infants in early months of life (Stewart CJ et al., Nature 2018;562:583-8; Ho NT et al., Nature Communications 2018;9:4169).

A suitable and healthy gut microbiota is a key factor in the development of the mucosal immune system of the infant. It is known that, amongst other ingredients, non-digestible carbohydrates (prebiotics) in particular can affect the promotion of particular microbiota.

Human breast milk is an immunologically active fluid, which contains an abundance of structurally diverse oligosaccharides, known collectively as human milk oligosaccharides (HMOs), which may support immune function through several probable mechanisms. These include a prebiotic effect resulting in the development and maintenance of a healthy gut microbiome, a key factor in the development of the mucosal immune system (Bode et al,. Glycobiology 2012;22(9):1147-1162). HMOs may also function as soluble decoy receptors in the gut, protecting the neonate from enteric pathogens (Newburg et al, Human milk glycans protect infants against enteric pathogens." Annual Review of Nutrition 2005;25:37-58) and may directly interact with gut epithelial cells yielding changes that could interfere with host-microbial interactions (Bode et al, 2012).

WO2009060073 from Nestec SA relates to the use of an oligosaccharide such as lacto-N-tetraose or lacto-N-neotetraose to promote the development in the first few weeks of the life of the infant of a beneficial intestinal microbiota comparable with that found in breastfed infants, especially an intestinal microbiota dominated by appreciable populations of Bifidobacterium and Lactobacillus species to the exclusion of other populations such as species Bacteroides, Clostridia and Streptococci.

WO2017021476 relates to a nutritional composition comprising at least one fucosylated oligosaccharide and at least one N-acetylated oligosaccharide for promoting or inducing a global gut microbiota that is closer to the one of infants fed exclusively with human breast milk, in comparison to infants fed with a conventional nutritional composition.

Borschel et al., Nutrients, 2018;10(3):289 relates to the comparison of growth of healthy term infants fed extensively hydrolysed protein and amino acid-based infant formulas
European Food safety authority, The EFSA Journal, 2015;13(7), 4183-1, assessed the safety of lacto-N-neotetraose as a novel food ingredient pursuant to Regulation (EC) No 258/97.

US2019209593 relates to a nutritional composition for infants or young children and their health effects, that comprise at least one fucosylated oligosaccharide and at least on eN-acetylated oligosaccharide, for use to prevent and/or treat infection in an infant or young child, by promoting and/or reducing specific microbiota communities in an infant or young child.

US2018333426 relates to paediatric nutritional compositions including galacto-oligosaccharide and human milk oligosaccharide or a precursor thereof, which advantageously modify the gut microbiome and improve select markers of immunity, brain structure, and gut function.

However, no solution is currently available to slow the above-mentioned premature shift towards an adult-type microbiome in formula-fed infants.

Accordingly, there remains a significant need for nutritional compositions, such as infant formulas and young-child formulas, that may be used to prevent or reduce the premature maturation of the gut microbiota, in particular infant or young-child formulas formulas that are effective in modulating maturation of the gut microbiome in cow's milk allergic infants.

There is a need to deliver such health benefits in these infants or young children in a manner that does not induce side effects, that is easy to deliver, and well accepted by the parents or health care practitioners.

### SUMMARY OF THE INVENTION

The inventors have surprisingly found that supplementation of infant formula with the human milk oligosaccharides (HMOs) 2'-fucosyllactose (2'FL) and lacto-N-neotetraose (LNnT) can advantageously be used to inhibit or reduce the premature shift towards an adult-type gut microbiome previously described in infants receiving no or only some breast milk.

In a controlled, double-blind, randomised interventional clinical trial it has surprisingly been found an hypoallergenic infant formula comprising the human milk oligosaccharides (HMOs) 2'-fucosyllactose (2'FL) and/or lacto-N-neotetraose (LNnT), leads to lower microbial diversity and reduced gut microbiota age at 12 months of age compared to a parallel hypoallergenic infant formula not comprising said HMOs.

Accordingly, in one aspect the invention provides an infant or young-child formula comprising the human milk oligosaccharides (HMOs) 2'-fucosyllactose (2'FL) and lacto-N-neotetraose (LNnT) for use in inhibiting or reducing premature maturation of the gut microbiota, wherein the infant formula is an extensively hydrolysed infant formula (eHF) or an amino acid-based infant formula (AAF), and the infant has cow's milk protein allergy.

In an embodiment, the invention provides an infant or young-child formula comprising the human milk oligosaccharides (HMOs) 2'-fucosyllactose (2'FL) and lacto-N-neotetraose (LNnT) for use in delaying maturation of the gut microbiota, wherein the infant formula is an extensively hydrolysed infant formula (eHF) or an amino acid-based infant formula (AAF), and the infant has cow's milk protein allergy.

In one embodiment, the invention provides an infant or young-child formula comprising the human milk oligosaccharides (HMOs) 2'-fucosyllactose (2'FL) and lacto-N-neotetraose (LNnT) for use inducing a microbiota that is less diverse at 12 months age compared the microbiota at 12 months age of an infant receiving a conventional infant formula not comprising 2'FL and LNnT, wherein the infant formula is an extensively hydrolysed infant formula (eHF) or an amino acid-based infant formula (AAF), and the infant has cow's milk protein allergy.

In one embodiment, the invention provides an infant or young-child formula comprising the human milk oligosaccharides (HMOs) 2'-fucosyllactose (2'FL) and lacto-N-neotetraose (LNnT) for use inducing a lower gut microbiota age at 12 months age, compared to an infant receiving a conventional infant formula not comprising 2'FL and LNnT, wherein the infant formula is an extensively hydrolysed infant formula (eHF) or an amino acid-based infant formula (AAF), and the infant has cow's milk protein allergy.

Surprisingly, the advantageous benefits of the infant or young-child formula of the invention, comprising the HMOs 2'FL and LNnT, on the gut microbiome are observed in infants at 12-months age despite the diversification of diet, with associated reduction in the proportion of the dietary intake provided by the formula of the invention.

In a preferred embodiment, the infant or young-child formula comprising the human milk oligosaccharides (HMOs) 2'-fucosyllactose (2'FL) and lacto-N-neotetraose (LNnT) according to the invention is consumed by the infant at least up to 12 months age.

In a preferred embodiment, the infant or young-child formula comprising the human milk oligosaccharides (HMOs) 2'-fucosyllactose (2'FL) and lacto-N-neotetraose (LNnT) according to the invention is the sole or predominant infant or young-child formula consumed by the infant at least up to 12 months age.

It can be especially used in providing a healthy growth, in providing a healthy immune system, in providing a healthy gut function and/or in preventing microbiota dysbiosis in infants or young children, and in infants or young children with cow's milk protein allergy.

The infant or young-child formula comprises 2'FL and LNnT. In an embodiment, the formula may comprise 0.5-3 g/L, 0.8-1.5 g/L, or about 1 g/L 2'FL, preferably, the formula comprises about 1 g/L 2'FL; and/or the formula may comprise 0.2-1 g/L, 0.5-0.8 g/L, or about 0.5 g/L LNnT, preferably the formula comprises about 0.5 g/L LNnT. Most preferably, the formula comprises about 1 g/L 2'FL and about 0.5 g/L LNnT.

The infant or young-child has cow's milk protein allergy.

In one embodiment, infant or young-child formula is a hypoallergenic formula. The hypoallergenic infant or young child formula is an extensively hydrolysed formula (eHF) or an amino acid-based formula (AAF). In one embodiment, the infant or young child formula is an eHF. In one embodiment, the infant or young child formula is an AAF. Preferably, the infant formula is an eHF.

At least about 95%, at least about 98%, at least about 99% or about 100% by weight of the peptides in the eHF may have a molecular mass of less than about 3000 Da. Preferably, there are no detectable peptides in the eHF about 3000 Da or greater in size.

At least about 90%, at least about 95%, at least about 98% or at least about 99% by weight of the peptides in the eHF may have a molecular mass of less than about 1500 Da. Preferably, at least about 99% of the peptides in the eHF have a molecular mass of less than about 1500 Da.

At least about 85%, at least about 90%, at least about 95%, at least about 98% or at least about 99% by weight of the peptides in the eHF may have a molecular mass of less than about 1200 Da. Preferably, at least 98% of the peptides by weight have a molecular mass of less than about 1200 Da.

At least about 45%, at least about 50%, 45-55%, or 50-54% by weight of the peptides in the eHF may be di- and tri-peptides. Preferably, about 51-53%, or more preferably, about 52% by weight of the peptides in the eHF are di- and tri-peptides.

At least about 45%, at least about 50%, 45-55%, or 50-54% by weight of the peptides in the eHF may have a molecular weight of between 240 and 600 Da. Preferably, about 51-53%, or more preferably about 52% by weight of the peptides in the eHF have a molecular weight of between 240 and 600 Da.

At least about 50%, at least about 60%, at least about 70%, at least about 80%, at least about 90%, or about 100% of the protein in the eHF may be whey protein. Preferably, the protein source is whey protein.

The eHF may comprise free amino acids. The free amino acids may be present in a concentration of 50% or less, 40% or less, 30% or less, or 25% or less by weight based on the total weight of amino acids. Preferably, the free amino acids are present in a concentration of 20-25%, 21-23%, or about 22% by weight based on the total weight of amino acids.

In one embodiment the infant formula is eHF comprising protein, carbohydrate and fat, wherein the eHF comprises about 2.4 g or less protein per 100 kcal, or the AAF comprises about 2.9g or less protein per 100kcal wherein the infant formula further comprises 2'-fucosyllactose (2'FL) and/or lacto-N-neotetraose (LNnT), and wherein about 30% or less by weight of the fat is medium chain triglycerides (MCTs).

In another embdoment the infant formula is an AAF comprising protein, carbohydrate and fat, and comprises about 2.8g or less protein per 100kcal, preferably 2.7g or less protein per 100kcal, more preferably 2.6 g or less protein per 100 kcal, wherein the AAF further comprises 2'-fucosyllactose (2'FL) and/or lacto-N-neotetraose (LNnT), and wherein about 30% or less by weight of the fat is medium chain triglycerides (MCTs). Suitably, the AAF may comprise 2.5 g or less protein per 100 kcal.

In an embodiment, the infant formula may comprise 1.8-2.4 g protein per 100 kcal, 2.1-2.3 g protein per 100 kcal, or 2.15-2.25 g protein per 100 kcal. Preferably the infant formula comprises about 2.2 g protein per 100 kcal.

In an embodiment, about 30% or less by weight, about 25% or less by weight, 20% or less by weight, 15% or less by weight, 10% or less by weight, 5% or less by weight, or 1% or less by weight of the fat in the infant or young-child formula may be medium chain triglycerides (MCTs). Preferably, the infant formula comprises no added MCTs.

In an embodiment the infant formula may comprise 9-14 g carbohydrate per 100 kcal and/or 4.0-6.0 g fat per 100 kcal.

### BRIEF DESCRIPTION OF THE FIGURES

**Figure 1** represents transition models showing temporal development from early to late Fecal Community Type (FCT) clusters comparing groups fed Test formula (HMO) **Fig 1** **A** or Control Formula **Fig 1** **B**.
**Figure 2** illustrates differences in alpha diversity in infants at 12 months age, between groups fed Test formula (HMO) or Control Formula, represented by gene richness and Shannon diversity.
**Figure 3** - Illustrates differences in FCT distribution at 12 months age, between groups fed Test formula (HMO) or Control Formula

### DETAILED DESCRIPTION OF THE INVENTION

Various preferred features and embodiments of the present invention will now be described by way of non-limiting examples.

As used herein, the following terms have the following meanings.

The term "infant" means a child under the age of 12 months (<12 month).

The expression "young child" means a child aged between one and less than three years (≥1 year to <3 years), also called toddler, also called toddler.

The expression "conventional infant or young-child formula" refers to standard synthetic nutritional compositions such as infant formula, follow-up milks or growing-up milks already found in the market.

In this context, the terms "microbial", "microflora", "microbiome" and "microbiota" can be used interchangeably.

In this context, the expressions "gut microbiota" ,"intestinal microbiota", "gut microbiome" can be used interchangeably.

A suitable and healthy gut microbiota is a key factor in the development of the mucosal immune system of the infant.

The expressions "down regulation" and "reduction" can be used interchangeably.

By the expressions "preventing" or "prevention", it is meant avoiding that a physical state, a condition or their consequences occurs and/or decreasing its incidence (i.e. reduction of the frequency).

By the expressions "treating" or "treatment", it is meant a decrease of the duration and/or of the severity of a physical state, a condition or their consequences.

The prevention and/or the treatment of a physical state, a condition or their consequences can occur during the treatment (i.e. during the administration of the composition of the present invention, either immediately after the start of the administration or some time after, e.g. some days or weeks after the start). But it can also encompass the prevention and/or the treatment later in life. The term "later in life" encompasses the effect after the termination of the intervention or treatment. The effect "later in life" can be from 1 week to several months, for example from 2 to 4 weeks, from 2 to 6 weeks, from 2 to 8 weeks, from 1 to 6 months or from 2 to 12 months.

The term "prebiotic" means non-digestible carbohydrates that beneficially affect the host by selectively stimulating the growth and/or the activity of healthy bacteria such as bifidobacteria in the colon of humans (Gibson GR, Roberfroid MB. Dietary modulation of the human colonic microbiota: introducing the concept of prebiotics. J Nutr. 1995;125:1401-12).

The term "probiotic" means microbial cell preparations or components of microbial cells with a beneficial effect on the health or well-being of the host. (Salminen S, Ouwehand A. Benno Y. et al. "Probiotics: how should they be defined" Trends Food Sci. Technol. 1999:10 107-10). The microbial cells are generally bacteria or yeasts.

The term "cfu" should be understood as colony-forming unit.

All percentages are by weight unless otherwise stated.

In addition, it must be noted that as used herein and in the appended claims, the singular forms "a", "an", and "the" include plural referents unless the context clearly dictates otherwise.

The terms "comprising", "comprises" and "comprised of" as used herein are synonymous with "including" or "includes"; or "containing" or "contains", and are inclusive or open-ended and do not exclude additional, non-recited members, elements or steps. The terms "comprising", "comprises" and "comprised of" also include the term "consisting of".

As used herein the term "about" means approximately, in the region of, roughly, or around. When the term "about" is used in conjunction with a numerical value or range, it modifies that value or range by extending the boundaries above and below the numerical value(s) set forth. In general, the terms "about" and "approximately" are used herein to modify a numerical value(s) above and below the stated value(s) by 10%.

The publications discussed herein are provided solely for their disclosure prior to the filing date of the present application. Nothing herein is to be construed as an admission that such publications constitute prior art to the claims appended hereto.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range.

### Formula

The term "infant formula" may refer to a foodstuff intended for particular nutritional use by infants during the first year of life and satisfying by itself the nutritional requirements of this category of person, as defined in European Commission Regulation (EU) 2016/127 of 25 September 2015. It also refers to a nutritional composition intended for infants and as defined in Codex Alimentarius (Codex STAN 72-1981) and Infant Specialities (incl. Food for Special Medical Purpose). The expression "infant formula" encompasses both "starter infant formula" and "follow-up formula" or "follow-on formula". In some embodiments, the infant formula is a preterm formula.

A "follow-up formula" or "follow-on formula" is given from the 6th month onwards. It constitutes the principal liquid element in the progressively diversified diet of this category of person.

The expression "growing-up milk" (or GUM) refers to a milk-based drink generally with added vitamins and minerals, that is intended for young children or children.

In a particular embodiment the nutritional composition of the present invention is a hypoallergenic formula. The expression "hypoallergenic formula" means an infant or young-child formula which is unlikely to cause allergic reactions.

The infant formula of the present invention is an extensively hydrolysed infant formula (eHF) or an amino acid-based infant formula (AAF). Preferably, the infant formula is an eHF.

The term "extensively hydrolysed infant formula" or "eHF" may refer to an infant formula comprising extensively hydrolysed protein. The eHF may be a hypoallergenic infant formula which provides complete nutrition for infants who cannot digest intact CMP or who are intolerant or allergic to CMP.

The term "amino acid-based infant formula" or "AAF" may refer to an infant formula comprising only free amino acids as a protein source. The AAF may contain no detectable peptides. The AAF may be a hypoallergenic infant formula which provides complete nutrition for infants with food protein allergy and/or food protein intolerance. For example, the AAF may be a hypoallergenic infant formula which provides complete nutrition for infants who cannot digest intact CMP or who are intolerant or allergic to CMP, and who may have extremely severe or life-threatening symptoms and/or sensitisation against multiple foods.

A "hypoallergenic" composition is a composition which is unlikely to cause allergic reactions. Suitably, the infant formula of the invention is tolerated by more than 90% of infants with CMPA. This is in line with the guidance provided by the American Academy of Pediatrics (Committee on Nutrition, 2000. Pediatrics, 106(2), pp.346-349). Suitably, the infant formula of the invention may not contain peptides which are recognized by CMP-specific IgE e.g. IgE from subjects with CMPA.

Infants can be fed solely with the infant formula or the infant formula can be used as a complement of human milk.

The infant formula of the invention may be in solid form (e.g. powder) or in liquid.

The liquid may be, for example, a concentrated liquid infant formula or a ready-to-feed infant formula. The infant formula may be in the form of a reconstituted infant formula (i.e. a liquid infant formula that has been reconstituted from a powdered form). The concentrated liquid infant formula is preferably capable of being diluted into a liquid composition suitable for feeding an infant, for example by the addition of water.

In one embodiment, the infant formula is in a powdered form. The powder is capable of being reconstituted into a liquid composition suitable for feeding an infant, for example by the addition of water.

The amount of the various ingredients can be expressed in g/100g of composition on a dry weight basis when it is in a solid form, e.g. a powder, or as a concentration in g/L of the composition when it refers to a liquid form (this latter also encompasses liquid composition that may be obtained from a powder after reconstitution in a liquid such as milk, water... , e.g. a reconstituted infant formula or a follow-on/follow-up formula or an infant cereal product or any other formulation designed for infant nutrition). They can also be expressed in g/100 kcal.

The infant formula may have an energy density of about 60-72 kcal per 100 mL, when formulated as instructed. Suitably, the infant formula may have an energy density of about 60-70 kcal per 100 mL, when formulated as instructed.

### Human milk oligosaccharides

The infant formula of the invention contains two or more human milk oligosaccharide (HMO).

Many different kinds of HMOs are found in the human milk. Each individual oligosaccharide is based on a combination of glucose, galactose, sialic acid (N-acetylneuraminic acid), fucose and/or N-acetylglucosamine with many and varied linkages between them, thus accounting for the enormous number of different oligosaccharides in human milk - over 130 such structures have been identified so far. Almost all of them have a lactose moiety at their reducing end while sialic acid and/or fucose (when present) occupy terminal positions at the non-reducing ends. HMOs can be acidic (e.g. charged sialic acid containing oligosaccharide) or neutral (e.g. fucosylated oligosaccharide).

The infant formula of the invention comprises 2'-fucosyllactose (2'FL) and lacto-N-neotetraose (LNnT).

The infant formula of the invention comprises 2'FL. In some embodiments, there is no other type of fucosylated oligosaccharide than 2'FL, i.e. the infant formula of the invention comprises only 2'FL as fucosylated oligosaccharide.

The 2'FL may be produced by biotechnological means using specific fucosyltransferases and/or fucosidases either through the use of enzyme-based fermentation technology (recombinant or natural enzymes) or microbial fermentation technology. In the latter case, microbes may either express their natural enzymes and substrates or may be engineered to produce respective substrates and enzymes. Alternatively, 2'FL may be produced by chemical synthesis from lactose and free fucose.

The infant formula of the invention comprises LNnT. In some embodiments, there is no other type of N-acetylated oligosaccharide than LNnT, i.e. the infant formula of the invention comprises only LNnT as N-acetylated oligosaccharide.

The LNnT may be synthesised chemically by enzymatic transfer of saccharide units from donor moieties to acceptor moieties using glycosyltransferases as described for example in US patent No. 5,288,637 and WO 96/10086. Alternatively, LNnT may be prepared by chemical conversion of Keto-hexoses (e.g. fructose) either free or bound to an oligosaccharide (e.g. lactulose) into N-acetylhexosamine or an N-acetylhexosamine-containing oligosaccharide as described in Wrodnigg, T.M.; Stutz, A.E. (1999) Angew. Chem. Int. Ed. 38:827-828. N-acetyllactosamine produced in this way may then be transferred to lactose as the acceptor moiety.

The infant formula of the present invention comprises an oligosaccharide mixture that comprises 2'FL and LNnT. In a preferred embodiment, the infant formula of the present invention comprises an oligosaccharide mixture that consists of 2'FL and LNnT. The infant formula of the invention may comprise only 2'FL as fucosylated oligosaccharide and only LNnT as N-acetylated oligosaccharide.

2'FL can be present in the infant formula according to the present invention in a total amount of 0.5-3 g/L such as 0.8-1.5 g/L of the infant formula (when formulated as instructed). In some embodiments, 2'-fucosyllactose may be in a total amount of 0.85-1.3 g/L of the infant formula, such as 0.9-1.25 g/L or 0.9-1.1 g/L or 1-1.25 g/L or 1-1.2 g/L of the infant formula (when formulated as instructed). Preferably, the infant formula (when formulated as instructed) comprises about 1 g/L 2'-fucosyllactose.

LNnT can be present in the infant formula according to the present invention in a total amount of 0.2-1 g/L such as 0.5-0.8 g/L of the infant formula (when formulated as instructed). In some embodiments, LNnT may be in a total amount of 0.5-0.75 g/L or 0.5-0.7 g/L or 0.5-0.6 g/L of the infant formula (when formulated as instructed). Preferably, the infant formula (when formulated as instructed) comprises about 0.5 g/L LNnT.

These different ranges can all be combined together.

Therefore in one embodiment of the present invention, the infant formula (when formulated as instructed) comprises 2'FL and LNnT wherein:
(i) 2'FL is in a total amount of 0.8-1.5 g/L of the infant formula; and/or
(ii) LNnT is in a total amount of 0.5-0.8 g/L of the infant formula.

In another embodiment the infant formula of the present invention (when formulated as instructed) comprises 2'FL and LNnT wherein:
(i) 2'FL is in a total amount of 0.9-1.25 g/L of the infant formula; and/or
(ii) LNnT is in a total amount of 0.5-0.7 g/L of the infant formula.

In another embodiment the infant formula of the present invention (when formulated as instructed) comprises 2'FL and LNnT wherein:
(i) 2'FL is in a total amount of 1-1.2 g/L of the infant formula; and/or
(ii) LNnT is in a total amount of 0.5-0.6 g/L of the infant formula.

In a preferred embodiment, the infant formula of the present invention (when formulated as instructed) comprises about 1 g/L 2'FL and about 0.5 g/L LNnT.

The infant formula of the present invention may comprise 0.075-0.5 g/100kcal, 0.1-0.3 g/100kcal, or 0.12-0.25 g/100kcal 2'FL and about 0.03-0.15 g/100kcal, 0.05-0.12 g/100kcal, or 0.05-0.1 g/100kcal LNnT. Preferably, the infant formula of the present invention comprises about 0.15 g/100kcal 2'FL and about 0.075 g/100kcal LNnT.

The 2'FL and the LNnT comprised in the infant formula according to the invention are typically present in a ratio 2'FL: LNnT of from 2.0:0.54 to 2.0:2.26, such as 2.0:0.76 to 2.0:1.8 or 2.0:0.8 to 2.0:1.4. In a particularly advantageous embodiment, this ratio is 2.0:1 or around 2.0:1.

### Protein

The term "protein" includes peptides and free amino acids. The protein content of the infant formula may be calculated by any method known to those of skill in the art. Suitably, the protein content may be determined by a nitrogen-to-protein conversion method. For example, as described in Maubois, J.L. and Lorient, D., 2016. Dairy science & technology, 96(1), pp.15-25. Preferably the protein content is calculated as nitrogen content × 6.25, as defined in European Commission Regulation (EU) 2016/127 of 25 September 2015. The nitrogen content may be determined by any method known to those of skill in the art. For example, nitrogen content may be measured by the Kjeldahl method.

### Protein concentration

eHFs typically contain 2.6-2.8 g protein per 100 kcal and AAFs typically contain 2.8-3.1 g protein per 100 kcal, to cover the needs of infants suffering gastrointestinal pathologies with severe malabsorption or infants requiring more proteins and calories to cover a higher metabolic rate.

The inventors have surprisingly shown that an eHF or an AAF with a lower protein content may support appropriate growth and development of allergic infants. Moreover, the inventors have surprisingly shown that the formula was safe and well-tolerated.

Accordingly, the eHF of the present invention comprises about 2.4 g or less protein per 100 kcal, or the AAF of the present invention comprises about 2.9g or less protein per 100kcal, preferably 2.8g or less protein per 100kcal. For example, the infant formula of the present invention may comprise about 2.3 g or less protein per 100 kcal, 2.25 g or less protein per 100 kcal, or 2.2 g or less protein per 100 kcal.

Suitably, the infant formula comprises about 1.8 g or more protein per 100 kcal. For example, the infant formula of the present invention may comprise about 1.86 g or more protein per 100 kcal, 1.9 g or more protein per 100 kcal, 2.0 g or more protein per 100 kcal, or 2.1 g or more protein per 100 kcal. Preferably, the infant formula comprises about 1.86 g or more protein per 100 kcal, in line with present EU regulations (EFSA NDA Panel, 2014. EFSA journal, 12(7), 3760).

The eHF of the present invention may comprise 1.8-2.4 g protein per 100 kcal, 1.86-2.4g protein per 100 kcal, 1.9-2.4 g protein per 100 kcal, 2.0-2.4 g protein per 100 kcal, 2.0-2.3 g protein per 100 kcal, 2.1-2.3 g protein per 100 kcal, or 2.15-2.25 g protein per 100 kcal.

The AAF of the present invention may comprise 1.8-2.9 g protein per 100 kcal, 1.9-2.8g protein per 100 kcal, 2.0-2.7 g protein per 100 kcal, 2.0-2.6 g protein per 100 kcal, 2.0-2.5 g protein per 100 kcal, 2.0-2.4 g protein per 100 kcal, 2.1-2.3 g protein per 100 kcal, or 2.15-2.25 g protein per 100 kcal.

Preferably the infant formula of the present invention comprises between 2.0 and 2.4 g protein per 100 kcal, for example 2.1-2.3 g protein per 100 kcal, or 2.15-2.25 g protein per 100 kcal
Preferably, the infant formula comprises about 2.2 g protein per 100 kcal.

### Protein source

The source of protein may be any source suitable for use in an infant formula. Suitably, the protein is cow's milk protein.

An extensively hydrolysed/hydrolysed whey-based formula may be more palatable than an extensively hydrolysed/hydrolysed casein-based formula and/or the subject may only be sensitised to casein protein. Suitably, therefore, more than about 50%, more than about 60%, more than about 70%, more than about 80%, more than about 90%, or about 100% of the protein is whey protein. Preferably, the protein source is whey protein.

The whey protein may be a whey from cheese making, particularly a sweet whey such as that resulting from the coagulation of casein by rennet, an acidic whey from the coagulation of casein by an acid, or the acidifying ferments, or even a mixed whey resulting from coagulation by an acid and by rennet. This starting material may be whey that has been demineralized by ion exchange and/or by electrodialysis and is known as demineralised whey protein (DWP).

The source of the whey protein may be sweet whey from which the caseino-glycomacropeptide (CGMP) has been totally or partially removed. This is called modified sweet whey (MSW). Removal of the CGMP from sweet whey results in a protein material with threonine and trytophan contents that are closer to those of human milk. A process for removing CGMP from sweet whey is described in EP 880902.

The whey protein may be a mix of DWP and MSW.

In some embodiments, the amount of casein in the infant formula is undetectable, for example less than 0.2 mg/kg. The amount of casein may be determined by any method known to those of skill in the art.

### Degree of hydrolysis

Hydrolysed proteins may be characterised as "partially hydrolysed" or "extensively hydrolysed" depending on the degree to which the hydrolysis reaction is carried out. Currently there is no agreed legal/clinical definition of Extensively Hydrolyzed Products according to the WAO (World Allergy Organization) guidelines for Cow's milk protein allergy (CMA) but there is agreement that according to the WAO that hydrolysed formulas have proven to be a useful and widely used protein source for infants suffering from CMA. In the current invention partially hydrolysed proteins are one in which 60-70% of the protein/peptide population has a molecular weight of less than 1000 Daltons, whereas extensively hydrolysed proteins are one in which at least 95% of the protein/peptide population has a molecular weight of less than 1000 Dalton. These definitions are currently used in the industry. Partially hydrolysed proteins are usually considered as hypoallergenic (HA) whereas extensively hydrolysed proteins are usually considered as non-allergenic.

In eHFs, the protein is "extensively hydrolysed", such that the eHFs may be tolerated by more than 90% of infants with CMPA.

Protein hydrolysates may have an extent of hydrolysis that is characterised by NPN/TN%, which refers to the non-protein nitrogen divided by the total nitrogen × 100. The non-protein nitrogen refers to amino nitrogen that is free to react with a reagent such as trinitrobenzenesulfonic acid (TNBS). NPN/TN% may be determined by any method known to those of skill in the art. For example, NPN/TN% may be measured as described in Adler-Nissen (Adler-Nissen, J. (1979) J. Agric. Food Chem. 27: 1256-1262). Suitably, the protein may have an NPN/TN% greater than 90%, greater than 95% or greater than 98%.

The extent of hydrolysis may also be determined by the degree of hydrolysis. The "degree of hydrolysis" (DH) is defined as the proportion of cleaved peptide bonds in a protein hydrolysate and may be determined by any method known to those of skill in the art. Suitably the degree of hydrolysis is determined by pH-stat, trinitrobenzenesulfonic acid (TNBS), o-phthaldialdehyde (OPA), trichloroacetic acid soluble nitrogen (SN-TCA), or formol titration methods. (Rutherfurd, S.M., 2010. Journal of AOAC International, 93(5), pp.1515-1522). The degree of hydrolysis (DH) of the protein can be more than 90, more than 95 or more than 98.

The extent of hydrolysis may also be determined by the peptide molecular mass distribution. The peptide molecular mass distribution may be determined by High performance size exclusion chromatography, optionally with UV detection (HPSEC/UV) (Johns, P.W., et al., 2011. Food chemistry, 125(3), pp.1041-1050). For example, the peptide molecular mass distribution may be a HPSEC peak area-based estimate determined at 205 nm, 214 nm or 220 nm. Suitably when the peptide molecular mass distribution is determined by HPSEC/UV, the "percentage of peptides by weight" that have a certain molecular mass may be estimated by the "fraction of peak area as a percentage of total peak area", that have the molecular mass, determined at 205 nm, 214 nm or 220 nm. Suitably, the extent of hydrolysis may be determined by the methods described in WO 2016/156077. Alternatively, the peptide molecular mass distribution may be determined by any method known to those of skill in the art, for example by sodium dodecyl sulphate-polyacrylamide gel electrophoresis (SDS-PAGE) (Chauveau, A., et al., 2016. Pediatric Allergy and Immunology, 27(5), pp.541-543).

Theoretically, to bind with cell membrane-bound IgE, peptides should be greater than about 1500 Da in size (approximately 15 amino acids) and to crosslink IgE molecules and to induce an immune response, they must be greater than about 3000 Da in size (approximately 30 amino acids) (Nutten, 2018. EMJ Allergy Immunol, 3(1), pp. 50-59).

Suitably, therefore, at least about 95%, at least about 98%, at least about 99% or about 100% of the peptides by weight in the eHF have a molecular mass of less than about 3000 Da. There may be no detectable peptides about 3000 Da or greater in size in the eHF.

Suitably, therefore, at least about 95%, at least about 98%, at least about 99% or about 100% of the peptides by weight in the eHF have a molecular mass of less than about 1500 Da. Preferably, at least 99% of the peptides by weight have a molecular mass of less than about 1500 Da. There may be no detectable peptides about 1500 Da or greater in size in the eHF.

Preferably, at least about 85%, at least about 90%, at least about 95%, at least about 98% or at least about 99% of the peptides by weight in the eHF have a molecular mass of less than about 1200 Da. More preferably, at least 95% or 98% of the peptides by weight in the eHF have a molecular mass of less than about 1200 Da.

Suitably, at least about 80%, at least about 85%, at least about 90%, or at least about 95% of the peptides by weight in the eHF have a molecular mass of less than about 1000 Da. Preferably, at least about 95% of the peptides by weight in the eHF have a molecular mass of less than about 1000 Da.

Preferably, the eHF of the present invention has no detectable peptides about 3000 Da or greater in size; and at least about 95% of the peptides by weight have a molecular mass of less than about 1200 Da.

Having a high proportion of di- and tri-peptides may improve nitrogen (protein) absorption, even in patients with gut impairment. PEPT1 is a dedicated facilitator transport route for small peptide absorption (e.g. di- and tri-peptides). In the first weeks of life, intestinal PEPT1 is important for nutritional intake, and later for diet transition following weaning.

Thus, at least about 30%, at least about 40%, or at least about 50% of the peptides by weight in the eHF may be di- and tri-peptides. Preferably, at least about 45%, at least about 50%, 45-55%, or 50-54% of the peptides by weight in the eHF are di- and tri-peptides. More preferably, about 51-53%, or most preferably, about 52% of the peptides by weight in the eHF are di- and tri-peptides.

Suitably, at least about 30%, at least about 40%, or at least about 50% of the peptides by weight in the eHF have a molecular mass of between 240 and 600 Da. Preferably, at least about 45%, at least about 50%, 45-55%, or 50-54% of the peptides by weight in the eHF have a molecular mass of between 240 and 600 Da. More preferably, about 51-53%, or most preferably, about 52% of the peptides by weight in the eHF have a molecular mass of between 240 and 600 Da.

The peptides in the eHF may have a median molecular weight of 300Da to 370Da, preferably 320Da to 360Da.

The principal recognised cow's milk allergens are alpha-lactalbumin (aLA), beta-lactoglobulin (bLG) and bovine serum albumin (BSA).

Suitably, therefore, the eHF may have non-detectable aLA content, for example about 0.010 mg/kg aLA or less; the eHF may have non-detectable bLG content, for example about 0.010 mg/kg bLG or less; and/or the eHF may have non-detectable BSA content, for example about 0.010 mg/kg BSA or less. Preferably, the eHF of the invention comprises no detectable amounts of aLA, bLG and BSA. The content of aLA, bLG and BSA may be determined by any method known to those of skill in the art, for example ELISA.

In preferred embodiments, the eHF of the present invention: has no detectable peptides about 3000 Da or greater in size; at least about 95% of the peptides by weight have a molecular mass of less than about 1200 Da; optionally at least about 45%, at least about 50%, or 45-55% of the peptides by weight have a molecular mass of between 240 and 600 Da and/or are di- or tri-peptides; the eHF of the present invention comprises about 1 g/L 2'-fucosyllactose and about 0.5 g/L lacto-N-neotetraose and/or about 0.15 g/100kcal 2'-fucosyllactose and about 0.075 g/100kcal lacto-N-neotetraose; and the eHF comprises no added MCT.

### Method of hydrolysis

Proteins for use in the infant formula of the invention may be hydrolysed by any suitable method known in the art. For example, proteins may be enzymatically hydrolysed, for example using a protease. For example, protein may be hydrolysed using alcalase (e.g. at an enzyme:substrate ratio of about 1-15% by weight and for a duration of about 1-10 hours). The temperature may range from about 40°C to 60°C, for example about 55°C. The reaction time may be, for example, from 1 to 10 hours and pH values before starting hydrolysis may, for example, fall within the range 6 to 9, preferably 6.5 to 8.5, more preferably 7.0 to 8.0.

Porcine enzymes, in particular porcine pancreatic enzymes may be used in the hydrolysis process. For example, WO9304593 A1 discloses a hydrolysis process using trypsin and chymotrypsin, which includes a two-step hydrolysis reaction with a heat denaturation step in between to ensure that the final hydrolysate is substantially free of intact allergenic proteins. The trypsin and chymotrypsin used in these methods are preparations produced by extraction of porcine pancreas.

WO2016156077A1 discloses a process for preparing a milk protein hydrolysate comprising hydrolysing a milk-based proteinaceous material with a microbial alkaline serine protease in combination with bromelain, a protease from Aspergillus and a protease from Bacillus.

### Free amino acids

The infant formula of the invention may comprise free amino acids.

The levels of free amino acids may be chosen to provide an amino acid profile that is sufficient for infant nutrition, in particular an amino acid profile that satisfies nutritional regulations (e.g. European Commission Directive 2006/141/EC).

For example, free amino acids may be incorporated in the eHF of the invention to supplement the amino acids comprised in the peptides.

In AAF the protein content of the infant formula is provided by free amino acis.

Example free amino acids for use in the infant formula of the invention include histidine, isoleucine, leucine, lysine, methionine, cysteine, phenylalanine, tyrosine, threonine, tryptophan, valine, alanine, arginine, asparagine, aspartic acid, glutamic acid, glutamine, glycine, proline, serine, carnitine, taurine and mixtures thereof.

Suitably, therefore, the free amino acids in the eHF may be present in a concentration of 50% or less, 40% or less, 30% or less, or 25% or less by weight based on the total weight of amino acids. Preferably, the eHF comprises 25% or less by weight of free amino acids based on the total weight of amino acids. More preferably, the free amino acids in the eHF are present in a concentration of 20-25%, 21-23%, or about 22% by weight based on the total weight of amino acids.

The free amino acids content may be determined by any method known of skill in the art. Suitably, the free amino acids content may be obtained by separation of the free amino acids present in an aqueous sample extract by ion exchange chromatography and photometric detection after post-column derivatization with ninhydrin reagent. Total amino acids content may be obtained by hydrolysis of the test portion in 6 mol/L HCl under nitrogen and separation of individual amino acids by ion-exchange chromatography, as described above.

In preferred embodiments, the eHF of the present invention: has no detectable peptides about 3000 Da or greater in size; at least about 95% of the peptides by weight have a molecular mass of less than about 1200 Da; optionally at least about 45%, at least about 50%, or 45-55% of the peptides by weight have a molecular mass of between 240 and 600 Da and/or are di- or tri-peptides, and/or 20-25%, 21-23%, or about 22% by weight based on the total weight of amino acids; the eHF of the present invention comprises about 1 g/L 2'-fucosyllactose and about 0.5 g/L lacto-N-neotetraose and/or about 0.15 g/100kcal 2'-fucosyllactose and about 0.075 g/100kcal lacto-N-neotetraose; and the eHF comprises no added MCT.

### Carbohydrate

The carbohydrate content of the infant formula of the invention is preferably in the range 9-14 g carbohydrate per 100 kcal.

The carbohydrate may be any carbohydrate which is suitable for use in an infant formula.

Example carbohydrates for use in the infant formula of the invention include lactose, saccharose, maltodextrin and starch. Mixtures of carbohydrates may be used.

In one embodiment, the carbohydrate content comprises maltodextrin. In one embodiment, at least about 20%, at least about 25%, at least about 30%, at least about 35%, at least about 40%, at least about 50%, at least about 60% or at least about 70% by weight of the total carbohydrate content is maltodextrin.

In one embodiment, the carbohydrate content comprises lactose. In one embodiment, at least about 20%, at least about 25%, at least about 30%, at least about 35%, at least about 40%, at least about 50%, at least about 60% or at least about 70% by weight of the total carbohydrate content is lactose.

In one embodiment, the carbohydrate comprises lactose and maltodextrin.

### Fat

The fat content of the infant formula of the invention is preferably in the range 4.0-6.0 g fat per 100 kcal.

The fat may be any lipid or fat which is suitable for use in an infant formula.

Example fats for use in the infant formula of the invention include sunflower oil, low erucic acid rapeseed oil, safflower oil, canola oil, olive oil, coconut oil, palm kernel oil, soybean oil, fish oil, palm oleic, high oleic sunflower oil and high oleic safflower oil, and microbial fermentation oil containing long chain, polyunsaturated fatty acids.

The fat may also be in the form of fractions derived from these oils, such as palm olein, medium chain triglycerides (MCT) and esters of fatty acids such as arachidonic acid, linoleic acid, palmitic acid, stearic acid, docosahexaenoic acid, linolenic acid, oleic acid, lauric acid, capric acid, caprylic acid, and caproic acid.

Further example fats include structured lipids (i.e. lipids that are modified chemically or enzymatically in order to change their structure). Preferably, the structured lipids are sn2 structured lipids, for example comprising triglycerides having an elevated level of palmitic acid at the sn2 position of the triglyceride. Structured lipids may be added or may be omitted.

Oils containing high quantities of preformed arachidonic acid (ARA) and/or docosahexaenoic acid (DHA), such as fish oils or microbial oils, may be added.

Long chain polyunsaturated fatty acids, such as dihomo-γ-linolenic acid, arachidonic acid (ARA), eicosapentaenoic acid and docosahexaenoic acid (DHA), may also be added.

The infant formula may comprise 2-20 mg ARA per 100 kcal, 5-15 ARA per 100 kcal, or about 10 mg ARA per 100 kcal and/or 2-20 mg DHA per 100 kcal, 5-15 DHA per 100 kcal, or about 10 mg DHA per 100 kcal. Preferably, the infant formula comprises about 10 mg ARA per 100 kcal and about 10 mg DHA per 100 kcal.

### Medium chain triglycerides (MCTs)

A high concentration of MCT may impair early weight gain. MCT is not stored and does not support fat storage. For instance, Borschel et al. have reported that infants fed formula without MCT gained significantly more weight between 1-56 days than infants fed formulas containing 50% of the fat from MCT (Borschel, M., et al., 2018. Nutrients, 10(3), p.289).

Thus, about 30% or less by weight of the fat may be medium chain triglycerides (MCTs) in the infant formula of the present invention.

In some embodiments, about 25% or less by weight, 20% or less by weight, 15% or less by weight, 10% or less by weight, 5% or less by weight, 4% or less by weight, 3% or less by weight, 2% or less by weight, 1% or less by weight, 0.5% or less by weight, or 0.1% or less by weight of the fat is medium chain triglycerides (MCTs).

In some embodiments, 0-30% by weight, 0-25% by weight, 0-20% by weight, 0-15% by weight, 0-10% by weight, 0-5% by weight, 0-4% by weight, 0-3% by weight, 0-2% by weight, 0-1% by weight, 0-0.5% by weight, or 0-0.1% by weight of the fat is medium chain triglycerides (MCTs).

Preferably, the infant formula comprises no added MCTs. Suitably, about 0% by weight of the fat is MCTs and/or the infant formula comprises no detectable MCTs. Suitably, the infant formula comprises no MCTs.

In preferred embodiments, the eHF of the present invention: has no detectable peptides about 3000 Da or greater in size; at least about 95% of the peptides by weight have a molecular mass of less than about 1200 Da; 45-55% of the peptides by weight have a molecular mass of between 240 and 600 Da; free amino acids are present in a concentration of 20-25% by weight based on the total weight of amino acid; and the eHF comprises no added MCT.

### Further ingredients

The infant formula of the invention preferably also contains all vitamins and minerals understood to be essential in the daily diet in nutritionally significant amounts. Minimum requirements have been established for certain vitamins and minerals.

Example vitamins, minerals and other nutrients for use in the infant formula of the invention include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chlorine, potassium, sodium, selenium, chromium, molybdenum, taurine and L-carnitine. Minerals are usually added in their salt form.

The infant formula of the invention may comprise one or more carotenoids.

The infant formula of the invention may also comprise at least one probiotic. The term "probiotic" refers to microbial cell preparations or components of microbial cells with beneficial effects on the health or well-being of the host. In particular, probiotics may improve gut barrier function.

Preferred probiotics are those which as a whole are safe, are L(+) lactic acid producing cultures and have acceptable shelf-life for products that are required to remain stable and effective for up to 24 months.

Examples of probiotic micro-organisms for use in the infant formula of the invention include yeasts, such as *Saccharomyces, Debaromyces, Candida, Pichia* and *Torulopsis;* and bacteria, such as the genera *Bifidobacterium, Bacteroides, Clostridium, Fusobacterium, Melissococcus, Propionibacterium, Streptococcus, Enterococcus, Lactococcus, Staphylococcus, Peptostrepococcus, Bacillus, Pediococcus, Micrococcus, Leuconostoc, Weissella, Aerococcus, Oenococcus* and *Lactobacillus.*

Specific examples of suitable probiotic microorganisms are: *Saccharomyces cereviseae, Bacillus coagulans, Bacillus licheniformis, Bacillus subtilis, Bifidobacterium bifidum, Bifidobacterium infantis, Bifidobacterium longum, Enterococcus faecium, Enterococcus faecalis, Lactobacillus acidophilus, Lactobacillus alimentarius, Lactobacillus casei* subsp. *casei, Lactobacillus casei Shirota, Lactobacillus curvatus, Lactobacillus delbruckii* subsp. *lactis, Lactobacillus farciminus, Lactobacillus gasseri, Lactobacillus helveticus, Lactobacillus johnsonii, Lactobacillus rhamnosus (Lactobacillus GG*), *Lactobacillus sake, Lactococcus lactis, Micrococcus varians, Pediococcus acidilactici, Pediococcus pentosaceus, Pediococcus acidilactici, Pediococcus halophilus, Streptococcus faecalis, Streptococcus thermophilus, Staphylococcus carnosus* and *Staphylococcus xylosus.*

The infant formula of the invention may also contain other substances which may have a beneficial effect such as prebiotics, lactoferrin, fibres, nucleotides, and nucleosides.

### Health Benefit

The infant or young child formula of the invention, comprising the human milk oligosaccharides (HMOs) 2'-fucosyllactose (2'FL) and lacto-N-neotetraose (LNnT), can advantageously be used to inhibit or reduce the premature shift towards an adult-type gut microbiome previously described in infants receiving no or only some breast milk.

The hypoallergenic infant formula comprising the human milk oligosaccharides (HMOs) 2'-fucosyllactose (2'FL) and/or lacto-N-neotetraose (LNnT), according to the invention can advantageously be used to induce lower microbial diversity and reduced gut microbiota age at 12 months of age compared to a parallel conventional (commercial) hypoallergenic infant formula not comprising said 2'FL and LNnT.

The infant or young-child formula of the present invention has a positive effect on the overall microbiota of the subject infants or young children: it inhibits or reduces premature ageing of the microbiota in the gut of the infants or young children fed with the nutritional composition of the present invention, compared to infants or young children fed predominantly or exclusively with a conventional nutritional composition not comprising said 2'FL and LNnT.

Surprisingly, the advantageous benefits of the infant or young-child formula of the invention, comprising the HMOs 2'FL and LNnT, on the gut microbiome are observed in infants at 12-months age despite the diversification of diet, with associated reduction in the proportion of the dietary intake of the infant provided by infant or young-child formula.

A suitable and healthy gut microbiota is a key factor in the development of the mucosal immune system of the infant.

In one aspect the invention provides an infant or young-child formula comprising the human milk oligosaccharides (HMOs) 2'-fucosyllactose (2'FL) and lacto-N-neotetraose (LNnT) for use in inhibiting or reducing premature maturation of the gut microbiota, wherein the infant formula is an extensively hydrolysed infant formula (eHF) or an amino acid-based infant formula (AAF), wherein the infant has cow's milk protein allergy.

In an embodiment the invention provides an infant or young-child formula comprising the human milk oligosaccharides (HMOs) 2'-fucosyllactose (2'FL) and lacto-N-neotetraose (LNnT) for use in delaying maturation of the gut microbiota, wherein the infant formula is an extensively hydrolysed infant formula (eHF) or an amino acid-based infant formula (AAF), wherein the infant has cow's milk protein allergy.

In an embodiment, the invention provides an infant or young-child formula comprising the human milk oligosaccharides (HMOs) 2'-fucosyllactose (2'FL) and lacto-N-neotetraose (LNnT) for use in delaying maturation of the gut microbiota towards an adult type gut microbiota, wherein the infant formula is an extensively hydrolysed infant formula (eHF) or an amino acid-based infant formula (AAF), wherein the infant has cow's milk protein allergy.

In one embodiment, the invention provides an infant or young-child formula comprising the human milk oligosaccharides (HMOs) 2'-fucosyllactose (2'FL) and lacto-N-neotetraose (LNnT) for use inducing a microbiota that is less diverse at 12 months age compared the microbiota at 12 months age of an infant receiving a conventional infant formula not comprising 2'FL and LNnT, wherein the infant formula is an extensively hydrolysed infant formula (eHF) or an amino acid-based infant formula (AAF), wherein the infant has cow's milk protein allergy.

In one embodiment, the invention provides an infant or young-child formula comprising the human milk oligosaccharides (HMOs) 2'-fucosyllactose (2'FL) and lacto-N-neotetraose (LNnT) for use inducing a lower gut microbiota age at 12 months age, compared to an infant receiving a conventional infant formula not comprising 2'FL and LNnT, wherein the infant formula is an extensively hydrolysed infant formula (eHF) or an amino acid-based infant formula (AAF), wherein the infant has cow's milk protein allergy.

Surprisingly, the advantageous benefits of the infant or young-child formula of the invention, comprising the HMOs 2'FL and LNnT, on the gut microbiome are observed in infants at 12-months age despite the diversification of diet, with associated reduction in the proportion of the dietary intake provided by the formula of the invention.

In a preferred embodiment the infant or young-child formula comprising the human milk oligosaccharides (HMOs) 2'-fucosyllactose (2'FL) and lacto-N-neotetraose (LNnT) according to the invention is consumed by the infant at least up to 12 months age.

In a preferred embodiment the infant or young-child formula comprising the human milk oligosaccharides (HMOs) 2'-fucosyllactose (2'FL) and lacto-N-neotetraose (LNnT) according to the invention is the sole or predominant infant or young-child formula consumed by the infant at least up to 12 months age.

In one embodiment inhibiting or reducing premature maturation of the gut microbiota means inducing a lower microbial diversity at 12 months age compared to an infant receiving conventional infant formula not comprising 2'FL and LNnT
In one embodiment inhibiting or reducing premature maturation of the gut microbiota means inducing a lower microbiota age at 12 months age compared to an infant receiving conventional infant formula not comprising 2'FL and LNnT
In one embodiment a lower microbiota age at 12 months age means having a gut microbiome enriched in early-type faecal community type (FCT) clusters, compared to an infant receiving an infant formula not comprising said 2'FL and LNnT.

In one embodiment a lower microbiota age at 12 months age means having a gut microbiome diminished in late-type faecal community type (FCT) clusters compared to an infant receiving an infant formula not comprising said 2'FL and LNnT.

"Early" type FCT are those FCT clusters generally associated with young breast-milk fed infants, e.g. infants up to 6 months age. In the context of the study described in the examples below early type FCT corresponds to the FCT clusters GC1, GC2 and GC3.

"Late" type FCT are those FCT clusters generally associated with older infants, children and adults. In the context of the study described in the examples below early type FCT corresponds to the FCT cluster GC5.

The infant or young-child formula of the invention can be especially used in providing a healthy growth, in providing a healthy immune system, e.g in preventing or reducing occurrence of infections, in providing a healthy gut function and/or in preventing microbiota dysbiosis in infants or young children, and in infants or young children with cow's milk protein allergy.

### Method of manufacture

The infant formula of the invention may be prepared in any suitable manner.

For example, the infant formula may be prepared by blending together the hydrolysed protein source, the carbohydrate source and the fat source in appropriate proportions. If used, the further emulsifiers may be included at this point. The vitamins and minerals may be added at this point but vitamins are usually added later to avoid thermal degradation. Any lipophilic vitamins, and emulsifiers may be dissolved in the fat source prior to blending. Water, preferably water which has been subjected to reverse osmosis, may then be mixed in to form a liquid mixture. Commercially available liquefiers may be used to form the liquid mixture. The liquid mixture may then be homogenised.

The liquid mixture may then be thermally treated to reduce bacterial loads. This may be carried out, for example, by means of steam injection, or using an autoclave or heat exchanger, for example a plate heat exchanger.

The liquid mixture may then be cooled and/or homogenised. The pH and solid content of the homogenised mixture may be adjusted at this point.

The homogenised mixture may then be transferred to a suitable drying apparatus such as a spray dryer or freeze dryer and converted to powder. If a liquid infant formula is preferred, the homogenised mixture may be sterilised, then aseptically filled into a suitable container or may be first filled into a container and then retorted.

### EXAMPLES

The invention will now be further described by way of Examples, which are meant to serve to assist one of ordinary skill in the art in carrying out the invention and are not intended in any way to limit the scope of the invention.

### Example 1 - Illustrative extensively hydrolysed infant formula

Below is an illustrative extensively hydrolysed infant formula according to the present invention. The eHF of the invention preferably contains all nutrients, vitamins and minerals understood to be essential in the daily diet in nutritionally significant amounts. Minimum requirements have been established for certain nutrients, vitamins and minerals.

| **Nutrient** | **Unit** | **per 100g** | **per 100kcal** | **per 100ml** |
|---|---|---|---|---|
| Energy | kcal | 506 | 100 | 67 |
| Fat | g | 26 | 5.1 | 3.4 |
| MCT | g | 0 | 0 | 0 |
| Available carbohydrates | g | 57 | 11 | 7.5 |
| Protein | g | 11.1 | 2.2 | 1.5 |
| 2'FL | g | 0.76 | 0.15 | 0.1 |
| LNnT | g | 0.38 | 0.075 | 0.05 |

### Example 2 - Effect of extensively hydrolysed infant formula supplemented with 2'FL and LNnT on gut microbiome development in infants with cow's milk protein allergy.

### Study design

The effects of an extensively hydrolysed infant formula (eHF) supplemented with 2'FL and LNnT on the faecal microbiome in infants with Cow's milk protein allergy (CMPA) was investigated in a controlled, double blind, randomized, multi-center, interventional clinical trial of 2 parallel formula fed groups.

Infants aged 0-6 months with CMPA were randomised to receive a lactose-containing commercial eHF (Althéra) , with or without 2'FL & LNnT, and a parallel eHF with reduced level of protein (Althéra 2.2) and comprising the two Human Milk Oligosaccharides (HMOs) 2'FL and LNnT (Test Formula) from enrolment to 12 months of age. The commercial eHF is currently approved as a food for special medical purposes (Regulation (EU) 2016/128).

The trial population was full-term infants with physician diagnosed CMPA as per standard clinical practice and with at least 2 symptoms per inclusion criterion. 130 infants completing 4 months of study formula intake were required.

The inclusion criteria were:
1. Full term infant (37 weeks ≤ gestation ≤ 42 weeks);
2. 2500g ≤ birth weight ≤ 4500g;
3. Having obtained the infant's parent's (or both parents' if required per country regulation) or legally authorized representative's (LAR) written informed consent;
4. Infant aged between birth and 6 months;
5. Exclusively formula-fed at time of enrolment or mothers of CMPA infant doing breastfeeding and independently elected before enrolment to exclusively formula feed; and
6. Infants with physician diagnosed (and untreated with extensively hydrolysed or amino acid infant formula) CMPA as per standard clinical practice and with at least 2 symptoms present from the following - Crying, Regurgitations, Liquid stools or Constipation, Skin atopic lesion, Urticaria or Respiratory symptoms. For diagnosis based on either a positive Ig E blood test, skin prick test, patch test or food challenge, only 1 symptom from above needs to be present.

The exclusion criteria were:
1. Congenital illness or malformation that may affect growth.
2. Demonstrated chronic malabsorption not due to CMPA.
3. Significant pre-natal and/or serious post-natal disease other than CMPA before enrolment (per investigator's medical decision).
4. Minor parent(s).
5. Infants whose parents or caregivers cannot be expected to comply with study procedures.
6. Currently participating or having participated in another clinical trial since birth.

The Test formula and the Control formula are shown below:

| | | **Test formula** | | | **Control formula** | | |
|---|---|---|---|---|---|---|---|
| **Nutrient** | **Unit** | **per 100g** | **per 100kcal** | **per 100ml** | **per 100g** | **per 100kcal** | **per 100ml** |
| Energy | kcal | 506 | 100 | 67 | 506 | 100 | 67 |
| Fat | g | 26 | 5.1 | 3.4 | 26 | 5.1 | 3.4 |
| MCT (Medium Chain Triglycerides) | g | 0 | 0 | 0 | 0 | 0 | 0 |
| Available Carbohydrates | g | 57 | 11 | 7.5 | 55.5 | 11 | 7.3 |
| Micronutrient/vitamin/ mineral mix | mg | 2350 | 465 | 310 | 2350 | 465 | 310 |
| 2'FL | g | 0.75 | 0.15 | 0.10 | - | - | - |
| LNnT | g | 0.38 | 0.075 | 0.05 | - | - | - |

In the Test formula and the Control formula:
- >99% by weight of the peptides had a molecular mass less than 3000 Da.
- >95% by weight of the peptides had a molecular mass less than 1200 Da.
- ~52% by weight of the peptides were di- and tri-peptides (peptides with a molecular mass of 600-240 Da).
- ~22.4% by weight of the total amino acids were free amino acids in the Test formula and Control formula.
- There was no detectable β-lactoglobulin (i.e. β-lactoglobulin content was less than 0.01 mg/kg).
- There was no detectable casein (i.e. casein content was less than 0.2 mg/kg).

Both formulas were in powder form, to be prepared as per instructions printed on the product label on the tins for oral intake by infants in amounts suitable for their weight, age and appetite.

Infants were given study formula until 4 month post-baseline at minimum (principal study period) and for as long as the infant requires per medical prescription (to maximum of 12 months of age).

Volume of feed required by the infant per day depended upon age, weight and appetite. Product was given *ad libitum* to the infant, although parents or caregivers followed guidelines printed on the label regarding appropriate volumes of feed to be offered per day and/or took advice from study staff.

Infants were attended up to 7 study visits: Baseline (enrolment), then every month until 4 months after baseline (+1, +2, +3, +4 months) and then 6 months after baseline. One additional final visit is planned when the infant will reach the age of 12 months old.

Randomization was a ratio of 1:1 per study formula group; and performed by minimization in Medidata balance. Stratification was by age at enrolment (0 to 60 days, 61 to 120 days, and above 120 days), gender, mode of delivery (vaginal or caesarean section). In case twins are enrolled, they were randomized to the same formula.

### Fecal Micobiota

Stool samples were collected from 132 infants (per-protocol set) at baseline (V0), 1 (V1) and 3 months (V3) from baseline, and at 12 months of age (V6).

Microbiome composition was profiled by metagenomics sequencing using the Metagenomics Species (MGS) approach [Nielsen, HB et al., Nature Biotechnology 2014;32:822], in which each MGS represents a known or novel clade at the species or subspecies level. Samples with similar microbiome composition were clustered into 5 faecal community types (FCT) and tracked within a transition model to analyse temporal development [Stewart CJ et al., Nature 2018;562:583-8].

Microbial richness and diversity (Shannon index) were compared between groups at each timepoint. Permutational ANOVA based on Bray-Curtis dissimilarity was used to compare the HMO effects on microbiome. Differences in taxonomical composition were assessed by an enrichment analysis at genus, family and phylum level.

### Study results

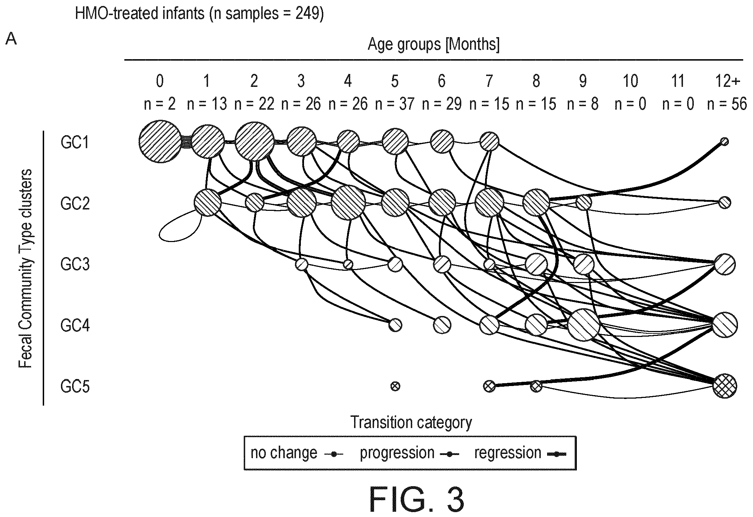

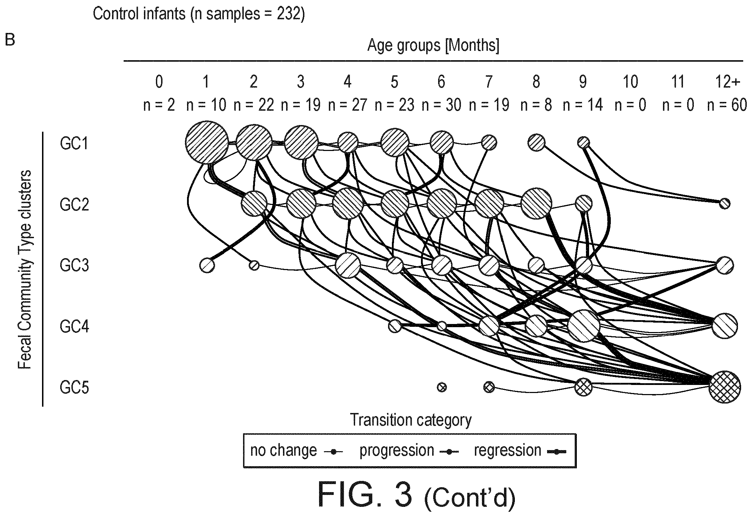

The microbiome trajectories showed a characteristic temporal development from "early" to "late" FCT (Figure 3), and from lower to higher alpha diversity. Microbiome development was strongly influenced by age. Significant differences between the study groups were not detected at V1 and V3, in part due to a wide age range at each timepoint. At 12 months of age (V6), the HMO-treated infants had a lower alpha diversity (Mann-Whitney U, MWU, p [richness] <0.003, p [diversity] <0.006) and were enriched in early-type FCT (2-sided MWU, p=0.014).

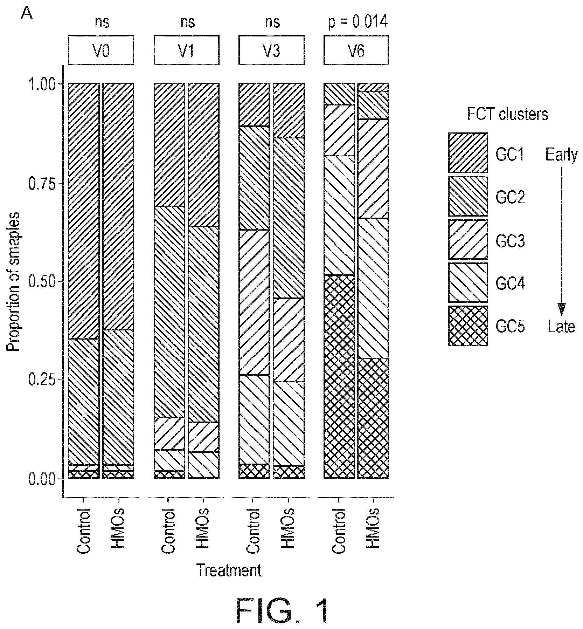

Figure 1A shows the differences in FCT distribution between the test and control groups stratified by visit. Groups were compared pairwise by MWU test, with FCT encoded as ordered factor (GC1 = 1, GC2 = 2, etc.). It shows that the test group "HMO group" (eHF supplemeted with 2'FL and LNnT) had higher prevalence of "early" FCT clusters, in particular GC1, GC2, GC3, compared to the control group, and lower prevalence of "late" FCT clusters, in particular GC5, compared to the control group. Thereby

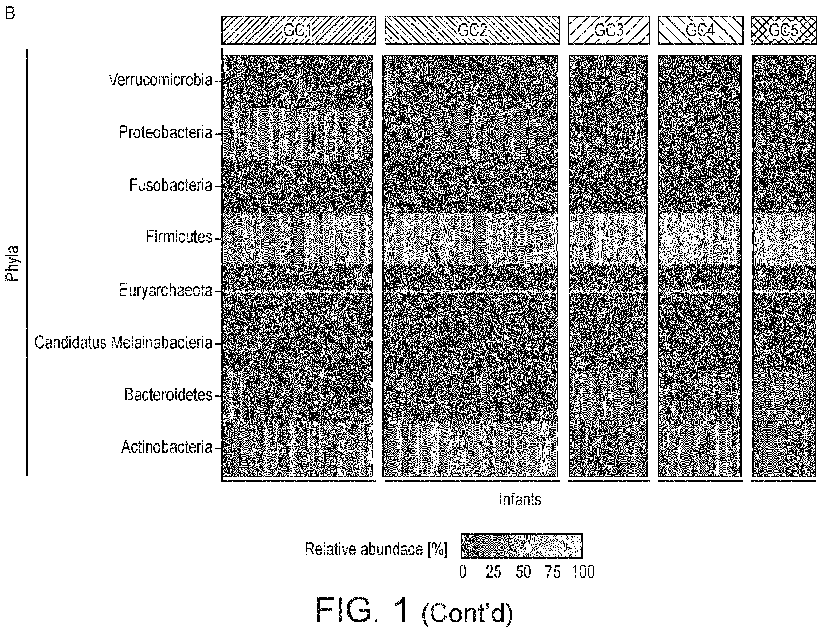

Figure 1B shows a taxon Taxonomical overview of each FCT cluster at phylum level showing mean abundance within each FCT (GC1-GC5) of the most abundant phyla.

The characterization of the FCT clusters obtained at genus level can be summarized as:
GC1 (Typical for newborn infants):
   Lowest alpha diversity
   Low abundance of bacteroidetes (phylum)
   High abundance of proteobacteria (phylum) and enterobacteriaceae (family)
   Low butyrate production
GC2 (Typical for infants between 1 month and 8 months):
   Intermediate alpha diversity
   High abundance of actinobacteria (phylum), and Bifidobacterium (genus)
   Low abundance of bacteroidetes (phylum)
GC3 (Typical for infants between 3 months and 1 year):
   Intermediate alpha diversity
   High abundance of firmicutes (phylum), lachnospiraceae (family), and lachnoclostridium (genus)
GC4 (Typical for infants > 6 months)
   Intermediate alpha diversity
   High abundance of firmicutes (phylum)
   Intermediate abundance of Bifidobacterium (genus)
GC5 (Typical for infants > 9 months)
   Highest alpha diversity
   High abundance of firmicutes (phylum) and faecalibacterium (genus)
   High abundance of bacteroidaceae (family)
   Higher butyrate production

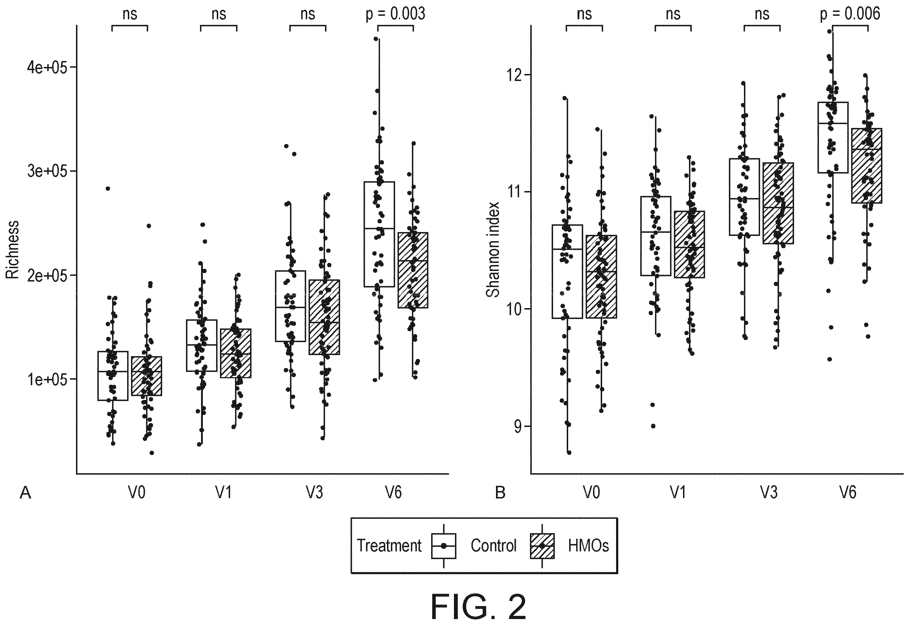

Figure 2 shows the gene richness (Fig. 2A) and Shannon diversity index (Fig. 2B) of the groups (i.e. how diverse the bacterial populations are in each sample). It illustrates that a 12 months age, the test group "HMO group" (eHF supplemeted with 2'FL and LNnT) infants had a lower alpha diversity than the control group.

Figure 3 shows a transition model illustrating temporal development from "early" to "late" FCT clusters. Transition models showing the progression of samples through each FCT, using all 481 samples. Node sizes represent the fraction of infants in a given cluster per age group (column) and line widths represent the fraction of transitions per age group (column). Figure 3 illustrates that the test group "HMO Group" (eHF supplemeted with 2'FL and LNnT) Fig 3A exhibited a slower temporal development from "early" towards "late" FCT clusters compared to the control group Fig 3B, corresponding to a lower microbiota age in the test group "HMO group" compared to the control group.

From the above results is can be concluded that the gut microbiome evolved with age in both study groups, reflecting changes in diet and environmental exposures. Feeding an HMO-supplemented EHF was associated with lower microbial diversity and reduced gut microbiota age at 12 months of age. Supplementation of eHF infant formula with 2'FL and LNnT was thus observed to slow the premature shift towards an adult-type gut microbiome previously described in infants receiving no or only some breast milk.

This observed persistence of the infants in an early stage of microbiome development, i.e. lower 'microbiome age', can be associated with the reduced rate of infections overall in the first year of life (previously published study results: Vandenplas Y et al., Oral poster presentation #1885 at EAACI Digital Congress, June 2020.), with the greatest reduction observed in the frequency of upper respiratory tract infections.

### Example 3 - Illustrative amino acid-based infant formula

Below is an illustrative amino acid-based infant formula according to the present invention. The AAF of the invention preferably contains all nutrients, vitamins and minerals understood to be essential in the daily diet in nutritionally significant amounts. Minimum requirements have been established for certain nutrients, vitamins and minerals.

| **Nutrient** | **Unit** | **per 100g** | **per 100kcal** | **per 100ml** |
|---|---|---|---|---|
| Energy | kcal | 503 | 100 | 70 |
| Fat | g | 25 | 5 | 3.5 |
| MCT | g | 0 | 0 | 0 |
| Available carbohydrates | g | 57 | 11.4 | 7.9 |
| Protein | g | 11 | 2.2 | 1.5 |
| 2'FL | g | 0.70 | 0.14 | 0.10 |
| LNnT | g | 0.36 | 0.07 | 0.05 |

Although the invention has been disclosed in connection with specific preferred embodiments, it should be understood that the invention as claimed should not be unduly limited to such specific embodiments. Indeed, various modifications of the disclosed modes for carrying out the invention, which are obvious to the skilled person are intended to be within the scope of the following claims.

## Claims

1. An infant or young-child formula comprising the human milk oligosaccharides (HMOs) 2'-fucosyllactose (2'FL) and lacto-N-neotetraose (LNnT) for use in (i) inhibiting or reducing premature maturation of the gut microbiota, and/or (ii) delaying maturation of the gut microbiota in an infant or young child, and/or (iii) inducing a microbiota that is less diverse at 12 months age compared the microbiota at 12 months age of an infant receiving a conventional infant formula not comprising 2'FL and LNnT, and/or (iv) inducing a lower gut microbiota age at 12 months age, compared to an infant receiving a conventional infant formula not comprising 2'FL and LNnT, wherein the infant formula is an extensively hydrolysed infant formula (eHF) or an amino acid-based infant formula (AAF), wherein the infant has cow's milk protein allergy.

2. An infant or young-child formula for use according to claim 1, wherein a lower microbiota age at 12 months age means having a gut microbiome enriched in early-type faecal community type (FCT) clusters, compared to an infant receiving an infant formula not comprising said 2'FL and LNnT.

3. An infant or young-child formula for use according to claim 1, wherein a lower microbiota age at 12 months age means having a gut microbiome diminished in late-type faecal community type (FCT) clusters compared to an infant receiving an infant formula not comprising said 2'FL and LNnT.

4. An infant formula for use according to any one of claims 1 to 3, wherein the infant formula comprises 0.5-3 g/L, 0.8-1.5 g/L, or about 1 g/L 2'FL, preferably wherein the infant formula comprises about 1 g/L 2'FL.

5. An infant formula for use according to any one of claims 1 to 4, wherein the infant formula comprises 0.2-1 g/L, 0.5-0.8 g/L, or about 0.5 g/L LNnT, preferably wherein the infant formula comprises about 0.5 g/L LNnT.

6. An infant formula for use according to any one of claims 1 to 5, wherein the infant formula comprises about 1 g/L 2'FL and about 0.5 g/L LNnT.

7. An infant formula for use according to any one of claims 1 to 6, wherein the infant formula is an eHF and wherein the infant formula comprises 1.8-2.4 g protein per 100 kcal, preferably between 2.0 and 2.4g protein per 100kcal, preferably 2.1-2.3 g protein per 100 kcal, or 2.15-2.25 g protein per 100 kcal.

8. An infant formula for use according to any one of claims 1 to 6, wherein the infant formula is an AAF, and wherein the infant formula comprises 1.8-2.9g protein per 100kcal, preferably 1.9-2.8g protein per 100 kcal, preferably 2.0-2.7 g protein per 100kcal, more preferably 2.0-2.6 g protein per 100 kcal, or 2.0-2.4 g per 100kcal.

9. An infant formula for use according to any one of claims 1 to 8, wherein the infant formula comprises about 2.2 g protein per 100 kcal.

10. An infant formula for use according to any one of claims 1 to 9, wherein about 25% or less by weight, 20% or less by weight, 15% or less by weight, 10% or less by weight, 5% or less by weight, or 1% or less by weight of the fat in the infant formula is medium chain triglycerides (MCTs).

11. An infant formula for use according to any one of claims 1 to 10, wherein the infant formula comprises no added MCTs.

12. An infant formula for use according to any one of claims 1 to 11, wherein the infant formula comprises 9-14 g carbohydrate per 100 kcal and/or 4.0-6.0 g fat per 100 kcal.

## Patentansprüche

1. Säuglings- oder Kleinkindanfangsnahrung, umfassend die humanen Milch-Oligosaccharide (HMOs) 2'-Fucosyllactose (2'FL) und Lacto-N-neotetraose (LNnT), zur Verwendung bei (i) einem Hemmen oder einem Verringern von vorzeitiger Reifung der Darmmikrobiota und/oder (ii) einem Verzögern der Reifung der Darmmikrobiota bei einem Säugling oder Kleinkind und/oder (iii) einem Induzieren einer Mikrobiota, die im Alter von 12 Monaten weniger vielfältig ist, verglichen mit der Mikrobiota im Alter von 12 Monaten eines Säuglings, der eine herkömmliche Säuglingsanfangsnahrung ohne 2'FL und LNnT erhält, und/oder (iv) dem Induzieren eines niedrigeren Darmmikrobiota-Alters im Alter von 12 Monaten, verglichen mit einem Säugling, der eine herkömmliche Säuglingsanfangsnahrung ohne 2'FL und LNnT erhält, wobei die Säuglingsanfangsnahrung eine extensiv hydrolysierte Säuglingsanfangsnahrung (eHF) oder eine auf Aminosäuren basierende Säuglingsanfangsnahrung (AAF) ist, wobei der Säugling eine Kuhmilchproteinallergie aufweist.

2. Säuglings- oder Kleinkindanfangsnahrung zur Verwendung nach Anspruch 1, wobei ein niedrigeres Mikrobiota-Alter im Alter von 12 Monaten bedeutet, dass ein Darmmikrobiom mit Clustern des frühen Fäkalgemeinschaftstyps (FCT) angereichert ist, verglichen mit einem Säugling, der eine Säuglingsanfangsnahrung erhält, die das 2'FL und das LNnT nicht umfasst.

3. Säuglings- oder Kleinkindanfangsnahrung zur Verwendung nach Anspruch 1, wobei ein niedrigeres Mikrobiota-Alter im Alter von 12 Monaten bedeutet, dass das Darmmikrobiom in Clustern des späten Fäkalgemeinschaftstyps (FCT) reduziert ist, verglichen mit einem Säugling, der eine Säuglingsanfangsnahrung erhält, die das 2'FL und das LNnT nicht umfasst.

4. Säuglingsanfangsnahrung nach einem der Ansprüche 1 bis 3, wobei die Säuglingsanfangsnahrung 0,5-3 g/l, 0,8-1,5 g/l oder etwa 1 g/l 2'FL umfasst, vorzugsweise wobei die Säuglingsanfangsnahrung etwa 1 g/l 2'FL umfasst.

5. Säuglingsanfangsnahrung für die Verwendung nach einem der Ansprüche 1 bis 4, wobei die Säuglingsanfangsnahrung 0,2-1 g/l, 0,5-0,8 g/l, oder etwa 0,5 g/l LNnT umfasst, vorzugsweise wobei die Säuglingsanfangsnahrung etwa 0,5 g/l LNnT umfasst.

6. Säuglingsanfangsnahrung für die Verwendung nach einem der Ansprüche 1 bis 5, wobei die Säuglingsanfangsnahrung etwa 1 g/l 2'FL und etwa 0,5 g/l LNnT umfasst.

7. Säuglingsanfangsnahrung zur Verwendung nach einem der Ansprüche 1 bis 6, wobei die Säuglingsanfangsnahrung eine eHF ist und wobei die Säuglingsanfangsnahrung 1,8-2,4 g Protein pro 100 kcal, vorzugsweise zwischen 2,0 und 2,4 g Protein pro 100 kcal, vorzugsweise 2,1-2,3 g Protein pro 100 kcal oder 2,15-2,25 g Protein pro 100 kcal umfasst.

8. Säuglingsanfangsnahrung zur Verwendung nach einem der Ansprüche 1 bis 6, wobei die Säuglingsanfangsnahrung eine AAF ist und wobei die Säuglingsanfangsnahrung 1,8-2,9 g Protein pro 100 kcal, vorzugsweise 1,9-2,8 g Protein pro 100 kcal, vorzugsweise 2,0-2,7 g Protein pro 100 kcal, stärker bevorzugt 2,0-2,6 g Protein pro 100 kcal oder 2,0-2,4 g pro 100 kcal umfasst.

9. Säuglingsanfangsnahrung zur Verwendung nach einem der Ansprüche 1 bis 8, wobei die Säuglingsanfangsnahrung etwa 2,2 g Protein pro 100 kcal umfasst.

10. Säuglingsanfangsnahrung zur Verwendung nach einem der Ansprüche 1 bis 9, wobei etwa 25 Gew.-% oder weniger, 20 Gew.-% oder weniger, 15 Gew.-% oder weniger, 10 Gew.-% oder weniger, 5 Gew.-% oder weniger oder 1 Gew.-% oder weniger des Fetts in der Säuglingsanfangsnahrung mittelkettige Triglyceride (MCTs) sind.

11. Säuglingsanfangsnahrung zur Verwendung nach einem der Ansprüche 1 bis 10, wobei die Säuglingsanfangsnahrung keine zugesetzten MCTs umfasst.

12. Säuglingsanfangsnahrung zur Verwendung nach einem der Ansprüche 1 bis 11, wobei die Säuglingsanfangsnahrung 9-14 g Kohlenhydrat pro 100 kcal und/oder 4,0-6,0 g Fett pro 100 kcal umfasst.

## Revendications

1. Préparation pour nourrisson ou jeune enfant comprenant les oligosaccharides de lait humain (HMO), 2'-fucosyllactose (2'FL) et lacto-N-néotétraose (LNnT) pour utilisation dans (i) l'inhibition ou la réduction de la maturation prématurée du microbiote intestinal, et/ou (ii) le retard de la maturation du microbiote intestinal chez un nourrisson ou un jeune enfant, et/ou (iii) l'induction d'un microbiote moins diversifié à l'âge de 12 mois par rapport au microbiote à l'âge de 12 mois d'un nourrisson recevant une préparation pour nourrissons conventionnelle ne comprenant pas de 2'FL et de LNnT, et/ou (iv) l'induction d'un microbiote intestinal moins âgé à l'âge de 12 mois, comparé à celui d'un nourrisson recevant une préparation pour nourrisson conventionnel ne comprenant pas de 2'FL et de LNnT, dans laquelle la préparation pour nourrisson est une préparation pour nourrisson à hydrolyse extensive (eHF) ou une préparation pour nourrisson à base d'acides aminés (AAF), et dans laquelle le nourrisson est allergique aux protéines de lait de vache.

2. Préparation pour nourrisson ou jeune enfant pour utilisation selon la revendication 1, dans laquelle un âge inférieur du microbiote à l'âge de 12 mois signifie avoir un microbiome intestinal enrichi en groupes de type de communauté fécale précoce (FCT), par rapport à un nourrisson recevant une préparation pour nourrisson ne comprenant pas lesdits 2'FL et LNnT.

3. Préparation pour nourrisson ou jeune enfant pour utilisation selon la revendication 1, dans laquelle un âge inférieur du microbiote à l'âge de 12 mois signifie avoir un microbiome intestinal diminué dans les groupes de type de communauté fécale tardive (FCT) par rapport à un nourrisson recevant une préparation pour nourrisson ne comprenant pas lesdits 2'FL et LNnT.

4. Préparation pour nourrisson pour utilisation selon l'une quelconque des revendications 1 à 3, dans laquelle la préparation pour nourrisson comprend 0,5 à 3 g/L, 0,8 à 1,5 g/L, ou environ 1 g/L de 2'FL, de préférence, dans laquelle la préparation pour nourrisson comprend environ 1 g/L de 2'FL.

5. Préparation pour nourrisson pour utilisation selon l'une quelconque des revendications 1 à 4, dans laquelle la préparation pour nourrisson comprend 0,2 à 1 g/L, 0,5 à 0,8 g/L, ou environ 0,5 g/L de LNnT, de préférence, dans laquelle la préparation pour nourrisson comprend environ 0,5 g/L de LNnT.

6. Préparation pour nourrisson pour utilisation selon l'une quelconque des revendications 1 à 5, dans laquelle la préparation pour nourrisson comprend environ 1 g/L de 2'FL et environ 0,5 g/L de LNnT.

7. Préparation pour nourrisson pour utilisation selon l'une quelconque des revendications 1 à 6, dans laquelle la préparation pour nourrisson est un eHF et dans laquelle la préparation pour nourrisson comprend 1,8 à 2,4 g de protéines pour 100 kcal, de préférence, entre 2,0 et 2,4 g de protéines pour 100 kcal, de préférence, 2,1 à 2,3 g de protéines pour 100 kcal, ou 2,15 à 2,25 g de protéines pour 100 kcal.

8. Préparation pour nourrisson pour utilisation selon l'une quelconque des revendications 1 à 6, dans laquelle la préparation pour nourrisson est un AAF, et dans laquelle la préparation pour nourrisson comprend 1,8 à 2,9 g de protéines pour 100 kcal, de préférence, 1,9 à 2,8 g de protéines pour 100 kcal, de préférence, 2,0 à 2,7 g de protéines pour 100 kcal, puis préférablement, 2,0 à 2,6 g de protéines pour 100 kcal, ou 2,0 à 2,4 g pour 100 kcal.

9. Préparation pour nourrisson pour utilisation selon l'une quelconque des revendications 1 à 8, dans laquelle la préparation pour nourrisson comprend environ 2,2 g de protéines pour 100 kcal.

10. Préparation pour nourrisson pour utilisation selon l'une quelconque des revendications 1 à 9, dans laquelle environ 25 % ou moins en poids, 20 % ou moins en poids, 15 % ou moins en poids, 10 % ou moins en poids, 5 % ou moins en poids, ou 1 % ou moins en poids des matières grasses de la préparation pour nourrisson sont des triglycérides à chaîne moyenne (TCM).

11. Préparation pour nourrisson pour utilisation selon l'une quelconque des revendications 1 à 10, dans laquelle la préparation pour nourrisson ne comprend pas de TCM ajoutés.

12. Préparation pour nourrisson pour utilisation selon l'une quelconque des revendications 1 à 11, dans laquelle la préparation pour nourrisson comprend 9 à 14 g de glucides pour 100 kcal et/ou 4,0 à 6,0 g de matières grasses pour 100 kcal.
